(19) Europäisches Patentamt
European Patent Office
Office européen des brevets

(11) Publication number: **0 288 176 A1**

# (12) EUROPEAN PATENT APPLICATION

(21) Application number: **88303004.1**

(22) Date of filing: **05.04.88**

(51) Int. Cl.[4]: **C07C 103/66 , C07C 102/00 , C07D 307/85 , A61K 31/195 , A61K 31/34 , A61K 37/26**

(30) Priority: **15.04.87 JP 92894/87**

(43) Date of publication of application: **26.10.88 Bulletin 88/43**

(84) Designated Contracting States: **CH DE FR GB LI**

(71) Applicant: **AJINOMOTO CO., INC.**
**5-8, Kyobashi 1-chome, Chuo-ku**
**Tokyo 104(JP)**

(72) Inventor: **Fukushima, Koji**
**No. 2-23-5-3, Hijirigaoka**
**Tama-shi Tokyo(JP)**
Inventor: **Seto, Yosiko**
**No. 1-2-3-406, Natsumidai**
**Funabashi-shi Chiba-ken(JP)**
Inventor: **Amino, Yusuke c/o Central Research Laboratories**
**Ajinomoto Co. Inc. No. 1-1 Suzuki-cho**
**Kawasaki-ku Kawasaki-shi**
**Kanagawa-ken(JP)**
Inventor: **Eto, Hirozumi c/o Central Research Laboratories**
**Ajinomoto Co. Inc. No. 1-1 Suzuki-cho**
**Kawasaki-ku Kawasaki-shi**
**Kanagawa-ken(JP)**
Inventor: **Eguchi, Chikahiko Central Research Laboratories**
**Ajinomoto Co. Inc. No. 1-1 Suzuki-cho**
**Kawasaki-ku Kawasaki-shi**
**Kanagawa-ken(JP)**
Inventor: **Toi, Koji Central Research Laboratories**
**Ajinomoto Co. Inc. No. 1-1 Suzuki-cho**
**Kawasaki-ku Kawasaki-shi**
**Kanagawa-ken(JP)**

(74) Representative: **Armitage, Ian Michael et al**
**MEWBURN ELLIS & CO. 2/3 Cursitor Street**
**London EC4A 1BQ(GB)**

EP 0 288 176 A1

## (54) Tyrosine derivatives and use thereof.

(57) Novel O-alkyl tyrosine derivatives of low toxicity have the effect of enhancing absorption of pharmaceutically active substances such as peptides, administered by mouth or as a suppository: compounds of the invention are those of formula (I) below, esters and salts thereof wherein $R^1$ is a lower alkyl group optionally substituted with

an alkyloxy group; X is CO or $SO_2$; -Y-is either a direct bond, lower alkylene group, substituted or unsubstituted vinylene group, or a group expressed as $-CHR^3$-O-or -O-$CHR^3$-; $R^2$ is a substituted or unsubstituted phenyl group, naphthyl group or 2-benzo-furanyl group; $R^3$ is either hydrogen or lower alkly group; or in the formula (I). $R_2$-Y-X denotes N-benzyloxycarbonyl phenylalanyl, N-benzyloxycarbonyl-4-halogenophenylalanyl, or N-(m-methoxycinnamoyl) phenylalanyl group.

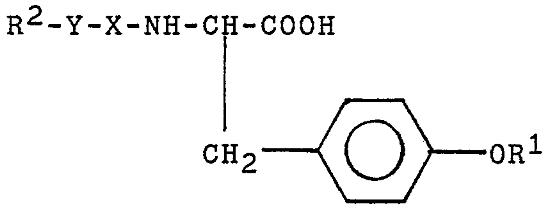

(I)

## TYROSINE DERIVATIVES AND USE THEREOF

The present invention relates to novel tyrosine derivatives and an absorption promoting agent containing the same as its active ingredient, and aims at imparting or improving absorption of drugs such as insulin.

There are known in the prior art such absorption promoting agents as phenylalanine derivatives (Jap. Pat. Appln. Laid Open No.190926/1984 ), benzoyl piperazine derivatives (Jap. Pat. Appln. Laid Open No. 5115/1984 ) and hydroxy benzoate derivatives (Biochimica et Biophysica Acta. 775 (1984), 269-271). Tyrosine derivatives according to the present invention differ from them in the nucleus substance. None of the existing tyrosine derivatives is known to have an absorption promoting effect.

Polypeptide based drugs are not absorbed in the intestinal tract as they are inactivated by proteolytic enzymes in digestive juice when perorally administered or because of their high molecular weight, and therefore are administered only by injections. Injections, however, cause physical as well as psychological pains in patients who are on prolonged therapy. There is an urgent need for development of absorption promoting agents with low toxicity in recent years.

The present invention relates to novel tyrosine derivatives, represented by the following general formula (I), esters and salts thereof, and a novel absorption imparting or promoting agent containing as an active ingredient at least one kind of said derivatives and non-toxic salts thereof;

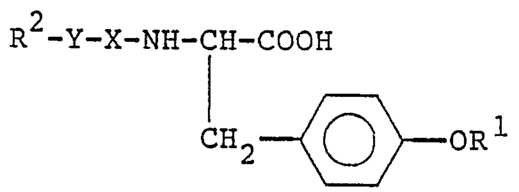

(I)

wherein $R^1$ is a lower alkyl group having 1 to 5 carbon atoms such as methyl and ethyl, or a lower alkyl group substituted with an alkyloxy group having 1 to 7 carbon atoms such as methoxy group; X is CO or $SO_2$; -Y-is either a direct bond, lower alkylene group, substituted or unsubstituted vinylene group such as fluorine and/or chorine substituted vinylene group or a group expressed as $-CHR^3$-or $-O-CHR^3$-; $R^2$ is a substituted or unsubstituted phenyl, naphthyl or 2-benzofuranyl group (substitution with, for example, halogen such as chlorine, nitro group, lower alkyl group such as methyl, ethyl, and trifluoromethyl or lower alkyloxy group such as methoxy); $R^3$ is hydrogen or lower alkyl group; or, in the formula (I), $R^2$-Y-CO is N-benzyloxycarbonyl phenylalanyl, N-benzyloxycarbonyl-4-halogeno (e.g. fluoro) phenylalanyl, or N-(m-methoxycinnamoyl)phenylalanyl group.

Said tyrosine derivatives or salts thereof that are pharmaceutically acceptable are effective in causing or promoting peroral or non-peroral (e.g. rectal) absorption of pharmaceutically active substances when such substances are administered in combination with the title compounds.

Derivatives of O-alkyl tyrosine expressed by said formula (I) are specific, and O-alkyl tyrosine per se or N-acetyl-O-alkyl tyrosine and lower alkylamides of acids represented by the formula (I) are not useful as an absorption promoting agent.

Although O-alkyl tyrosine derivatives represented by the formual (I) are novel substances, they can be prepared by the conventional N-acylation reaction, salt manufacturing process or manufacturing process to be described below.

Derivatives represented by the following general formula (II) and esters or salts thereof are novel compounds;

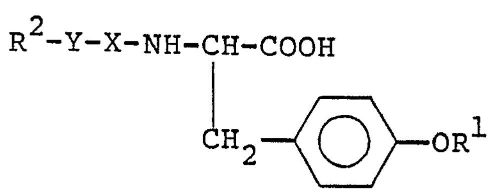

(II)

(wherein $R^1$, $R^2$ and Y are identical as those mentioned above).

Asymmetric carbon atoms are present in O-alkyl tyrosine derivatives represented by the formulae (I) and (II), and they may be in any of L-form, D-form, or DL-form depending on the combination of substituent

groups.

Said O-alkyl tyrosine derivatives may be in the form of salt. For example, they may be metallic salt such as of sodium, potassium, lithium and calcium, organic base such as ammonia, dicyclohexylamine, N-methyl-D-glucosamine, basic amino acid such as lysine and arginine are included. For medicinal uses, pharmaceutically acceptable salts are preferably used.

Said O-alkyl tyrosine derivatives or pharmaceutically acceptable salts thereof may be perorally or non-perorally (e.g. rectally) administered combined with pharmaceutically active substance as an agent to promote absorption. In the case of insulin, for example, they are effective in promoting absorption in peroral or rectal administration, and in suppressing trypsin and/or chymotrypsin that are proteolytic enzyme to decompose insulin.

Among insulin therapies for diabetic patients, in particular, peroral or rectal administration has not yet reached a practical stage, and development of an agent for promoting absorption of insulin with higher safety under prolonged continuous use is demanded in clinical medicine. The present invention is expected to be highly useful.

Toxicity of O-alkyl tyrosine derivatives is low; each of $LD_{50}$ in mice perorally administered of O-methyl-N-(4-methylcinnamoyl)-L-tyrosine (S-1), O-methyl-N-($\alpha$-fluoro-4-methylcinnamoyl)-L-tyrosine (S-5), O-methyl-N-($\beta$-chloro-4-methylcinnamoyl)-L-tyrosine (S-9) is 3.0 g/kg or higher.

Said pharmaceutically active substances may include polypeptides or derivatives thereof in which two or more of hydrophobic amino acid residual groups capable of assuming a conformation which allows interactions of non-covalent bond type with the adjuvant (O-alkyl tyrosine derivatives or the salt thereof) are present in proximity or in aggregate with one another at more than one location, and analogous substances of these two groups. More specifically, the following substances may be used: those in which an aggregate of hydrophobic amino acid residual groups is present at the surface of water soluble spherical protein (insulin, insulin-like growth factor I (IGF I), insulin-like growth factor II (IGF II), pancreatic polypeptide, etc.); cyclic peptides in which two or more hydrophobic amino acid residual groups are present adjacent to or proximal with one another; those in which two or more of hydrophobic amino acid residual groups are present in proximity or in aggregate at more than one locations in the preferential conformation which is formed in the co-existence of the absorption promoting agent of the present invention in an aqueous solution.

The absorption promoting agent according to the present invention is preferably used in an amount ranging from 0.1 to 2,000 mg, more preferably 0.2 to 500 mg, per 25 units of the drug, for example, insulin. The absorption promoting agent and the pharmaceutically active substance may be used in the form of tablets, capsules, elixirs, microcapsules or suspensions.

O-alkyl tyrosine derivatives of the present invention can be administered together with said drug to a patient requiring treatment in a dosage range of, for example, 0.1 to 1,000 mg, generally several times a day, in a total daily dosage of 0.2 to 2,000 mg. The dosage varies according to graveness of the disease, the bodyweight of the patient and other factors known by those skilled in the art.

Such typical combined uses mentioned above may be effected by formulating the substances into pharmaceutical compositions as described below. About 0.2 to 500 mg of the tyrosine derivatives of the present invention, physiologically acceptable salt compounds or mixtures thereof are blended into customary units demanded by generally accepted pharmaceutical practice together with physiologically acceptable vehicles, carriers, excipients, binders, antiseptics, stabilizers, flavorings, etc. The amount of active substance to be mixed in these compositions or preparations is adjusted in such a way as to give an appropriate dose of the prescribed range.

Specific materials that can be incorporated into tablets, capsules, etc. include the following; binders such as tragacanth, gum arabic, cornstarch or gelatin; excipients such as microcrystalline cellulose; swelling agents such as cornstarch, pregellatinized starch and arginic acid; lubricants such as magneisum stearate; sweetners such as sucrose, lactose or saccharin; and flavorings such as peppermint, oil from Gaultheria adenothrix Maxim or cherry. If the unit preparation is in a form of capsule, liquid carrier such as oil in addition to the above-mentioned materials may also be contained. Various other types of materials may be included as a coating agent or a material to change the physical unit of preparations. For example, shellac and/or sugar may be used for coating tablets. Syrup or elixir may be used as an active compound; sucrose as a sweetner; methylparaben or propylparaben as an antiseptic; and colors, cherry or orange flavorings as flavors.

In the case of insulin, it is particularly preferable to prepare it as an enteric preparation. For example, 8% aqueous solution of hydroxyphenylmethyl cellulose is used as a precoating agent, 10% aqueous solution of hydroxypropylmethyl cellulose phthalate and 3% aqueous solution of polyacetyne as coating agents to prepare enteric preparations by the known methods.

Specific examples of the tyrosine derivatives according to the present invention are given below:

S-1: O-methyl-N-(4-methylcinnamoyl)-L-tyrosine
S-2: O-ethyl-N-(4-methylcinnamoyl)-L-tyrosine
S-3: O-(2-methoxyethyl)-N-(4-methylcinnamoyl)-L-tyrosine
S-4: O-methyl-N-(α-fluorocinnamoyl)-L-tyrosine
S-5: O-methyl-N-(α-fluoro-4-methycinnamoyl)-L-tyrosine
S-6: O-ethyl-N-(α-fluroro-4-methylcinnamoyl)-L-tyrosine
S-7: O-methyl-N-(α-chloro-4-methylcinnamoyl)-L-tyrosine
S-8: O-methyl-N-(α-chloro-4-methylcinnamoyl)-D-tyrosine
S-9: O-methyl-N-(β-chloro-4-methylcinnamoyl)-L-tyrosine
S-10: O-methyl-N-(β-chloro-4-methylcinnamoyl)-D-tyrosine
S-11: O-methyl-N-(6-methylbenzo[b]furan-2-carbonyl)-L-tyrosine
S-12: O-methyl-N-(5-methylbenzo[b]furan-2-carbonyl)-L-tyrosine

The invention will now be described in more detail by way of the following examples.

## Examples

### Example 1: Preparation of Compounds S-1 through S-4

In a mixture of 200 ml water and 270 ml of acetone were dissolved 83.8 g of L-tyrosine and 20.8 g of sodium hydroxide, and the resulting mixture was added dropwise concurrently for 30 minutes with 100 ml of acetone solution containing 55.7 g of 4-methylcinnamoyl chloride prepared routinely under cooling at 0°C from thionyl chloride, and 100 ml of 3.5N hydroxide solution. The reaction solution was stirred for another 30 minutes at room temperature and added with hydrochloric acid to render the same acidic. The organic layer obtained after extraction with ethyl acetate was washed with saturated saline and dried with anhydrous magnesium sulfate. The residue obtained after distillation under reduced pressure was recrystallized using ethyl acetate-petroleum ether to obtain 75.0 g of N-(4-methylcinnamoyl)-L-tyrosine (yield: 74.8%).

In a mixture containing 100 ml each of water and acetone, 25.0 g of N-(4-methylcinnamoyl)-L-tyrosine and 11.3 g of sodium hydroxide were dissolved, and the resultant solution was added with 28.7 ml of methyl iodide and stirred for 5 hours at 50°C. Excess methyl iodide and acetone were distilled off under reduced pressure, and the reaction mixture was then added with 4.2 g of sodium hydroxide, 50 ml of water and 100 ml of acetone, and stirred for 30 minutes at room temperature. The reaction solution was made acidic with high concentration hydrochloric acid, and added with 200 ml of water. Precipitated crystals were filtered and washed with water, followed by recrystallization using acetone-water to obtain 22.1 g of the target substance S-1 in crystals (yield: 84.8%).

Compounds S-2 through S-4 were prepared similarly as above.

### Example 2: Preparation of Compounds S-5 through S-8, S-11, and S-12

In a mixture containing 200 ml of water and 100 ml of acetone, 52.2 g of N-(benzyloxycarbonyl)-L-tyrosine and 24.4 g of sodium hydroxide were dissolved, and the resultant solution was added with 61.8 ml of methyl iodide and stirred for 5 hours at 50°C. Excess methyl iodide and acetone were distilled off under reduced pressure, and resultant solution was then added with 10.5 g of sodium hydroxide and 100 ml of acetone and stirred for 30 minutes at room temperature. The resultant solution was added with 200 ml of water, and made acidic with high concentration hydrochloric acid. Precipitated crystals were filtered and washed with water to obtain crude crystals of O-methyl-N-(benzyloxycarbonyl)-L-tyrosine.

Crystals thus obtained were dissolved in 500 ml of methanol, added with 20 ml of acetic acid, 50 ml of water and 5.0 g of 5% palladium carbon, and stirred for 3 days in a hydrogen atmosphere under normal pressure at room temperature. Palladium carbon was removed by filtration, and further washed off using 100 ml of 2N hydrochloric acid solution. The filtrate was concentrated to about 200 ml under reduced pressure and washed with 100 ml of ethyl acetate. The aqueous layer was neutralized with 10N sodium

hydroxide solution to precipitate crystals, and the crystals were filtered, washed with water and dried under reduced pressure to obtain 19.2 g of O-methyl-L-tyrosine (yield: 59.3%).

To a mixed solution containing 300 ml of methanol and 100 ml of thionyl chloride maintained at 0°C was added 19.2 g of O-methyl-L-tyrosine and stirred for 30 minutes at 60°C. After distilling off the solvent under reduced pressure, the reaction solution was washed with ether to obtain 24.1 g of hydrochloride of O-methyl-L-tyrosine-methylester (yield: 99.6%).

In 200 ml of dioxane were suspended 5.6 g of α-fluoro-4-methylcinnamic acid and 7.7 g of hydrochloride of O-methyl-L-tyrosine-methylester. The reaction solution was maintained at 0°C, added with 4.4 ml of triethylamine, 5.7 g of HOBt, 7.7 g of DCC, stirred over-night at room temperature, further added with 4.0 ml of acetic acid, and stirred again for 2 hours at room temperature. The insoluble substance was removed by filtration, and the reaction solution was concentrated under reduced pressure. The residue was dissolved in 250 ml of ethyl acetate, washed with 5% $NaHCO_3$ solution, 0.5N hydrochloric acid solution and saturated saline, and dried with anhydrous magnesium sulfate. After distilling off the solvent under reduced pressure, the residue was separated and purified by silica gel chromatography. From the eluation using ethyl acetate-n-hexane (2 : 1), 11.4 g of crystals of O-methyl-N-(α-fluoro-4-methylcinnamoyl)-L-tyrosine methylester were obtained.

In a mixed solvent containing 50 ml of acetone and 25 ml of water was suspended 11.4 g of said methylester, added with 23.0 ml of 2N sodium hydroxide solution and stirred for 30 minutes at room temperature. The reaction solution was added with 150 ml of water, made acidic with high concentration hydrochloric acid and filtered to separate precipitated solids, which were washed with water. The solids were recrystallized with acetone-water to obtain 9.3 g of the object compound S-5 in crystals (yield: 83.9%).

The compounds S-6 through S-8, S-11 and S-12 were also prepared similarly as above.

Example 3: Preparation of Compounds S-9 and S-10

In a mixture of 60 ml water and 120 ml acetone were dissolved 6.9 g of O-methyl-L-tyrosine obtained in Example 2 and 1.6 g of sodium hydroxide. The resultant solution was added dropwise for 30 minutes at room temperature with acetone solution containing 6.6 g of β-chloro-4-methylcinnamoyl chloride prepared routinely using $SOCl_2$ under cooling at 0°C and 16.8 ml of 2N sodium hydroxide. The reaction solution was stirred for another 30 minutes at room temperature, added with 200 ml of water, and made acidic with high concentration hydrochloric acid. The organic layer obtained by extraction with ethyl acetate was washed with saturated saline and dried with anhydrous magnesium sulfate. The residue obtained after distillation under reduced pressure was recrystallized with ethyl acetate-petroleum ether to obtain 7.5 g of the object compound S-9 in crystals (yield: 65.8%).

The compounds S-10 was obtained similarly as above.

Physical properties of tyrosine derivatives prepared according to the present invention are shown in Table 1.

Table 1: Tyrosine Derivatives

| Example No. | Compound | Molecular | Melting point (°C) | Optical rotation |
|---|---|---|---|---|
| 1 | S-1 | $C_{20}H_{21}NO_4$ | 178 - 179 | $[\alpha]_D^{24}$ = -36.4° (C = 1.0 methanol) |
| " | S-2 | $C_{21}H_{23}NO_4$ | 184 - 185 | $[\alpha]_D^{25}$ = -36.3° (C = 1.0 methanol) |
| " | S-3 | $C_{22}H_{25}NO_5$ | 160 - 161 | $[\alpha]_D^{25}$ = -38.3° (C = 1.0 methanol) |
| " | S-4 | $C_{19}H_{18}NO_4F$ | 151 - 152 | $[\alpha]_D^{25}$ = -24.7° (C = 1.0 methanol) |
| 2 | S-5 | $C_{20}H_{20}NO_4F$ | 166 - 167 | $[\alpha]_D^{25}$ = -51.6° (C = 1.0 methanol) |
| " | S-6 | $C_{21}H_{22}NO_4F$ | 145.5 - 146.5 | $[\alpha]_D^{25}$ = -50.1° (C = 1.0 methanol) |
| " | S-7 | $C_{20}H_{20}NO_4Cl$ | 102 - 103 | $[\alpha]_D^{25}$ = +14.9° (C = 1.0 methanol) |
| " | S-8 | $C_{20}H_{20}NO_4Cl$ | 102 - 103 | $[\alpha]_D^{25}$ = -14.7° (C = 1.0 methanol) |
| 3 | S-9 | $C_{20}H_{20}NO_4Cl$ | 117 - 118 | $[\alpha]_D^{24}$ = -15.6° (C = 0.5 methanol) |
| " | S-10 | $C_{20}H_{20}NO_4Cl$ | 117 - 118 | $[\alpha]_D^{24}$ = +15.0° (C = 0.5 methanol) |
| 2 | S-11 | $C_{20}H_{19}NO_5$ | 155.5 - 156.5 | $[\alpha]_D^{25}$ = -111.7° (C = 1.0 methanol) |
| " | S-12 | $C_{20}H_{19}NO_5$ | 128 - 129 | $[\alpha]_D^{25}$ = -94.3° (C = 1.0 methanol) |

Example 4: Activity Test

The absorption promoting agents according to the present invention shown in Table 1 were suspended in 0.5% CMC - 0.05 M trisHCl buffer (pH 7.8), and mixed with insulin. The resultant mixture was administered perorally to female mice of ICR-CD-1 strain (aged 5 to 7 weeks) at 0.5 U insulin/10 g bodyweight and 3 mg of the above compound/10 g bodyweight. The percent decrease of blood glucose(%) and the degree of increase of blood insulin after predetermined periods of time were compared with those of the control group, and the results are shown in Table 2.

In the Table, the figures given in the upper rows denote 1) percent of decrease in blood glucose(%) and those in the lower rows in parentheses denote 2) degree of increase in blood insulin level. The symbol + means that there was no decrease in blood sugar and the symbol -means that there was no increase in blood insulin.

Table 2: Result of Activity Test

| Compound | 1) percent decrease in blood sugar (%)<br>2) degree of increase of blood insulin | | |
|---|---|---|---|
| | 15 min. | 30 min. | 60 min. |
| S-1 | 24.8<br>(3.7) | 25.8<br>(4.6) | 23.0<br>(2.6) |
| S-2 | 47.6<br>(8.2) | +<br>(-) | 3.3<br>(1.2) |
| S-3 | 26.0<br>(2.0) | 29.9<br>(2.1) | 10.7<br>(7.6) |
| S-4 | 62.6<br>(15.6) | 27.4<br>(2.3) | +<br>(2.9) |
| S-5 | 72.1<br>(27.0) | 3.6<br>(1.9) | +<br>(3.4) |
| S-6 | 29.1<br>(5.3) | +<br>(-) | +<br>(-) |
| S-7 | 28.6<br>(9.2) | +<br>(2.1) | +<br>(4.1) |
| S-8 | 51.8<br>(12.8) | 29.6<br>(4.7) | +<br>(2.7) |
| S-9 | 38.0<br>(2.2) | 11.6<br>(3.4) | 14.4<br>(-) |
| S-10 | 10.3<br>(2.2) | 8.7<br>(1.3) | +<br>(1.9) |
| S-11 | 44.1<br>(9.1) | 32.0<br>(3.3) | 20.4<br>(3.3) |
| S-12 | 38.5<br>(12.3) | 36.9<br>(9.2) | 33.2<br>(3.5) |

The above tables relate to peroral administration of the absorption promoting agents according to the present invention. However, they can be used combined with insulin as suppositories and the same effect can be achieved.

As has been described in detail, the present invention has made it practically possible to perorally or non-perorally (e.g. rectally) administer insulin, and the effect of the present invention is highly significant.

Example 5: Examples of Preparations

[Tablets]

To 30 ml of 0.05N hydrochloric acid was added with 0.577 g of swine insulin (15,000 Units, Zinc content, 0.5%), and the resultant solution was diluted to 300 ml with distilled water. After dissolving 6 g of the compound S-5 in 200 ml of 0.1N sodium hydroxide, pH was adjusted to 7.5 with 0.1N hydrochloric acid. The resultant solution was diluted to 600 ml by adding phosphoric acid buffer (0.02M, pH 7.5).

The compound S-5 solution was added dropwise with insulin solution at 20°C under violent agitation, and immediately after adjusting the pH value to 7.5, the mixture was freeze-dried.

25 mg of powder of said freeze-dried product, 82 mg of pregellatinized starch, 82 mg of microcrystalline cellulose and 1 mg of magnesium stearate were blended to prepare tablets. Using an aqueous solution of hydroxyphenylmethyl cellulose (8%) as a precoating agent, then aqueous solutions of hydroxypropylmethyl cellulose phthalate (10%) and of polyacetyl (3%) as coating agents, enteric coated tablets were prepared by the conventional process. Similar preparation was possible using the derivatives mentioned above in place of said O-alkyl tyrosine derivatives (S-5) with the pregellatinized starch, microcrystalline cellulose and magnesium stearate.

[Capsules]

To 250 ml of glacial acetic acid was added 30 g of the compound S-5, and the resultant mixture was heated and dissolved, cooled, and then added with 2 g of swine insulin (52,200 Units, zinc content, 0.5%) gradually while agitating. Acetic acid was distilled off under reduced pressure at the same temperature.

Residual solid thus obtained was added with 100 ml of n-hexane, pulverized, filtered, and washed. Adhered n-hexane was distilled off under reduced pressure. The resultant product was further dried under reduced pressure in the presence of solid sodium hydroxide.

Dry filling capsules containing 50 mg of active ingredient each were prepared.

Dry powder of the compound 50 mg
Lactose 149 mg
Magnesium stearate 1 mg
Capsule (size No. 1) 200 mg

Fifty mg of the dry powder was pulverized to obtain No. 60 powder, and lactose and magnesium stearate were passed through a No. 60 filter cloth over the dry powder and then thoroughly blended. The powder mixture was filled into No. 1 capsules of dried gelatin. Portion or all of the dry powder to be blended may be filled as enteric coated preparation mentioned above.

These results indicate that tyrosine derivatives according to the present invention, are useful as a pharmaceutically active substance, particularly as an agent to impart or promote absorption of insulin.

## Claims

1. A derivative of O-alkyl tyrosine represented by the following general formula (I), or an ester or salt thereof;

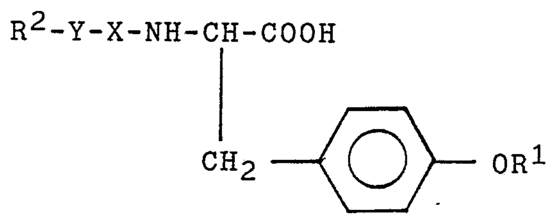

(I)

wherein $R^1$ is a lower alkyl group optionally substituted with an alkyloxy group; X is CO or $SO_2$; -Y-is either a direct bond, lower alkylene group, substituted or unsubstituted vinylene group, or a group expressed as $-CHR^3-O-$or $-O-CHR^3-$; $R^2$ is a substituted or unsubstituted phenyl group, naphthyl group or 2-benzo-furanyl group; $R^3$ is either hydrogen or lower alkyl group; or in the formula (I), $R^2$-Y-X denotes N-benzyloxycarbonyl phenylalanyl, N-benzyloxycarbonyl-4-halogenophenylalanyl, or N-(m-methoxycinnamoyl) phenylalanyl group.

2. A compound of claim 1 for use as a pharmaceutical absorption promoter.

3. A pharmaceutical absorption promoting agent comprising a compound of claim 1 and an inert diluent.

4. A pharmaceutical preparation comprising a compound of claim 1 arranged for simultaneous or sequential administration with a pharmaceutically active substance and providing enhanced absorption of said pharmaceutically active substance.

5. A pharmaceutical preparation according to claim 4 wherein the pharmaceutically active substance is a polypeptide or derivative thereof.

6. A pharmaceutical preparation according to claim 5 wherein the polypeptide is a water soluble spherical protein having an aggregate of hydrophobic amino groups, a cyclic peptide having two or more hydrophobic amino acid residues adjacent or proximal each others, or a peptide having two or more hydrophobic amino acid residues which are capable of being brought into proximity under the influence of a compound of claim 1.

7. A pharmaceutical preparation according to claim 6 wherein the polypeptide is insulin, insulin-like growth factor I (IGF I), insulin-like growth factor II (IGF II) or pancreatic polypeptide.

8. Use of a compound of claim 1 in the manufacture of a pharmaceutical preparation in which said derivative provides enhanced absorption of a pharmaceutically active substance.

9. A method for the production of a compound of claim 1 comprising

(i) N-acylation of a tyrosine derivative of formula (III)

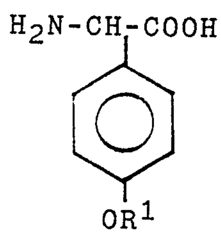

(III)

where $R^1$ is as defined above; or

(ii) O-alkylation of a compound of formula (IV)

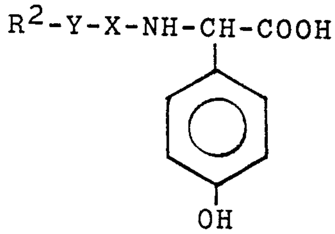

(IV)

optionally followed by

(iii) esterification, or

(iv) salt formation.

European Patent Office

# EUROPEAN SEARCH REPORT

Application number

EP 88303004.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | EP - A1 - 0 218 356 (AJINOMOTO) (15-04-1987)<br>* Claims; examples *<br>-- | 1-9 | C 07 C 103/66<br>C 07 C 102/00<br>C 07 D 307/85<br>A 61 K 31/195<br>A 61 K 31/34<br>A 61 K 37/26 |
| A | EP - A1 - 0 074 725 (KISSEI PHARMACEUTICAL)<br>* Claims *<br>---- | 1,9 | |
| | | | **TECHNICAL FIELDS SEARCHED (Int. Cl 4)** |
| | | | C 07 C 103/00<br>C 07 D 307/00 |

The present search report has been drawn up for all claims

| Place of search | Date of completion of the search | Examiner |
|---|---|---|
| VIENNA | 15-06-1988 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82